(19) Europäisches Patentamt
European Patent Office
Office européen des brevets

(11) EP 2 578 539 B1

## (12) EUROPEAN PATENT SPECIFICATION

(45) Date of publication and mention
of the grant of the patent:
**27.08.2014  Bulletin 2014/35**

(51) Int Cl.:
*C01G 49/08* [(2006.01)]     *C09C 1/24* [(2006.01)]

(21) Application number: **11008085.0**

(22) Date of filing: **06.10.2011**

(54) **Carbon encapsulated metal oxide nanocomposite, method for its preparation and its use in Li-ion batteries**

In Kohlenstoff eingekapseltes Metalloxid-Nanokomposit, Verfahren zu dessen Herstellung und dessen Verwendung in Li-Ionen-Akkus

Nanocomposite d'oxyde métallique encapsulé au carbone, son procédé de préparation et son utilisation dans les batteries au lithium-ion

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.04.2013  Bulletin 2013/15**

(73) Proprietor: **Karlsruher Institut für Technologie 76131 Karlsruhe (DE)**

(72) Inventors:
 • **Fichtner, Maximilian 68723 Oftersheim (DE)**
 • **Hahn, Horst 64342 Seeheim-Jugenheim (DE)**
 • **Prakash, Raju 76344 Eggenstein-Leopoldshafen (DE)**

(74) Representative: **Gärtner, Stephan Innovationsmanagement Postfach 3640 76021 Karlsruhe (DE)**

(56) References cited:
**US-A1- 2010 035 750     US-A1- 2011 104 553**

• VINEET PANCHAL ET AL: "Synthesis and characterization of carbon coated nanoparticles produced by a continuous low-pressure plasma process", JOURNAL OF NANOPARTICLE RESEARCH ; AN INTERDISCIPLINARY FORUM FOR NANOSCALE SCIENCE AND TECHNOLOGY, KLUWER ACADEMIC PUBLISHERS, DO, vol. 13, no. 9, 6 March 2011 (2011-03-06), pages 3825-3833, XP019937353, ISSN: 1572-896X, DOI: 10.1007/S11051-011-0305-3
• JULIANA C. TRISTÃO ET AL: "Facile preparation of carbon coated magnetic Fe3O4 particles by a combined reduction/CVD process", MATERIALS RESEARCH BULLETIN, vol. 46, no. 5, 1 May 2011 (2011-05-01), pages 748-754, XP55019054, ISSN: 0025-5408, DOI: 10.1016/j.materresbull.2011.01.008
• LIU H ET AL: "Magnetite/carbon core-shell nanorods as anode materials for lithium-ion batteries", ELECTROCHEMISTRY COMMUNICATIONS, ELSEVIER, AMSTERDAM, NL, vol. 10, no. 12, 1 December 2008 (2008-12-01), pages 1879-1882, XP025679956, ISSN: 1388-2481, DOI: 10.1016/J.ELECOM.2008.09.036 [retrieved on 2008-10-04]
• C.F. WANG ET AL: "Solid-Phase Synthesis of Carbon-Encapsulated Magnetic Nanoparticles", JOURNAL OF PHYSICAL CHEMISTRY C, vol. 111, no. 17, 3 May 2007 (2007-05-03), pages 6303-6307, XP55018980, ISSN: 1932-7447, DOI: 10.1021/jp0707283

EP 2 578 539 B1

## Description

### Technical field

The present invention provides a new $Fe_3O_4$ nanocomposite, consisting of carbon encapsulated magnetite nanoparticles and a convenient synthesis to obtain this nanocomposite. The nanocomposite is prepared in a one-step pyrolysis of an oxygen-containing organic iron compound without any added solvent or surfactant. The as prepared composite can be directly employed as anode material in a lithium ion cell. Moreover, the carbon encapsulated $Fe_3O_4$ nanoparticles can be used in biomedical or magnetic applications.

### Background

Magnetite ($Fe_3O_4$) is an attractive anode material for lithium-ion batteries (LIB) based on the conversion mechanism according to the reaction:

$$Fe_3O_4 + 8\ Li^+ + 8\ e^- \leftrightarrow 3\ Fe + 4\ Li_2O.$$

$Fe_3O_4$ is characterized by a theoretical capacity of 926 mAhg$^{-1}$, which is far beyond that of the graphite anode (372 mAhg$^{-1}$), eco-friendliness, natural abundance and high electronic conductivity ($2 \times 10^4$ Sm$^{-1}$). However, its application in practical lithium-ion batteries (LIB) is hindered due to its low rate performance arising from kinetic limitations, agglomerations and poor cycling stability resulting from significant volume expansion during the redox reaction.

To overcome these problems, Banov et al. (B. Banov, L. Ljutzkanov, I. Dimitrov, A. Trifonova, H. Vasilchina, A. Aleksandrova, A. Mochilov, B. T. Hang, S. Okada and J. I. Yamaki, 2008, J. Nanosci. Nanotech, 591-94) reported the synthesis of a $Fe_3O_4$ nanocomposite on a carbon matrix using by thermal decomposition of iron nitrate. This composite encompasses carbon particles with an average size of about 100-150 nm which are covered by small $Fe_3O_4$ crystallites of a mean size of 16 nm. The modified material yielded a specific capacity of about 400 mAh/g after 30 cycles, as anode in lithium batteries. However, the synthesis was achieved only by several successive steps including the preparation of the carbon support.

Liu et al. (H. Liu, G. Wang, J. Wang and D. Wexler, 2008, Electrochem. Commun. 1879-82) reported a synthesis of carbon coated magnetite ($Fe_3O_4$) core-shell by a multi-step hydrothermal method yielding $Fe_2O_3$ nanorods which were sintered together with citric acid as carbon source. The resulting carbon coated $Fe_3O_4$ nanorods have a diameter in the range of 30-50 nm and a length extending a few 100 nanometers. The as prepared $Fe_3O_4$/C core-shell nanorods exhibited an initial lithium storage capacity of 1120 mAh/g and a reversible capacity of 394 mAh/g after 100 cycles.

Muraliganth et al. (T. Muraliganth, A.V. Murugan and A. Manthiram, 2009, Chem.Commun., 7360-62) proposed a carbon-coating of $Fe_3O_4$-nanowires with glucose-derived carbon-rich polysaccharide in a two-step process by sonication and heating at 400°C for 3 h. However, $Fe_3O_4$-nanowires are complex to synthesize by a time and energy consuming procedure. The use of the $Fe_3O_4$-nanoparticles was not disclosed.

Wang et al. (S. Wang, J. Zhang and C. Chen, 2010, J. Power Sources, 195, 5379-81) have synthesized uniform submicron spheroids of $Fe_3O_4$ by a hydrothermal method. This material has been studied for its capacity as electrode material in Li-ion batteries. While having initially good characteristics, the material lacks of stability during the long term use in particular due to the volume changes and agglomeration of the particles.

In another example, $Fe_3O_4$-graphene (23 wt%) nanocomposite was prepared by a gas/liquid interface reaction (P. Lian, X. Zhu, H. Xiang, Z. Li, W. Yang and H. Wang, 2010, Electrochim. Acta, 834-40). The nanocomposite exhibited good capacity retention (99% after 90 cycles) with a large reversible capacity of 1048 mAh/g. However, the nanocomposite synthesis is a very complicated, multistep and time consuming process, required several work-up steps starting from iron nitrate and graphene sheets.

Song et al. (S. Song, R. Rao, H. Yang, H. Liu and A. Zhang, 2010, Nanotechnology, 185602(6pp)) reported a fabrication process of $Fe_3O_4$ nanoparticles with a size range of 4-8 nm by a spontaneous redox reaction between $Fe^{3+}$ and multi-walled carbon nanotubes (MWCNTs). In this process a multi-step preconditioning of the MWCNT is required. Moreover, no electrochemical studies were performed.

He at al. (H. He, L. Huang, J. S. Cai, X. M. Zheng and S. G. Sun, 2010, Electrochim. Acta 55, 1140-44) have reported the synthesis and electrochemical performance of nanostructured $Fe_3O_4$/carbon nanotube composite. In this complicated multi-step synthesis process, polyvinyl alcohol (PVA) was used as a hydrogen bond functionalizing agent to modify the multi-walled CNTs. The nanoparticles of $Fe_3O_4$ were then deposited along the sidewalls of the as- modified CNTs the chemical coprecipitation of $(NH_4)_2Fe(SO_4)$. $6H_2O$ and $NH_4Fe(SO4).12H_2O$ in the presence of CNTs in an alkaline solution. The final product was obtained by several purification steps including removal of ammonia, sulphate, unreacted, PVA etc.

In other report, Zhang et al. (M. Zhang, D. Lei, X. Yin, L. Chen, Q. Li, Y. Wang and T. Wang, 2010, J. Mater. Chem. 20, 5538-43) proposed the synthesis of magnetite/graphene composites by microwave irradiation method. In this method at first graphene oxide (GO) was prepared from graphite, then exfoliation was carried out by sonication of the GO. In the next step, the mixture of GO, $Fe(NO_3)_3$, urea and ascorbic acid was refluxed under ambient condition for 1h with a microwave heater. In addition, it requires several purification and heat treatment steps. The modified electrode material exhibited a reversible capacity of 650 mAh/g after 50 cycles.

Later, Zhou at al. (G. Zhou, D.-W. Wang, F. Li, L. Zhang, N. Li, Z.-S. Wu, L. Wen, G. Q. Lu and H.-M. Cheng, 2010, Chem. Mater. 22, 5306-13) reported the synthesis and electrochemical properties of graphene-wrapped $Fe_3O_4$ composite. The composite was synthesized by dispersing a graphene nanosheet and $FeCl_3$ in aqueous solution by sonication, followed by hydrolysis to obtain FeOOH/graphene sheet. After several purification steps, the resulting product was then reduced to give the desired nanocomposite by heat treatment under inert condition. This material was tested as anode in lithium batteries and obtained specific capacitiy of 1026 mAh/g after 30 cycles at a given current density of 35 mA/g.

Recently Ban et al. (C. Ban, Z. Wu, D. T. Gillaspie, L. Chen, Y. Yan, J. L. Blackbrurn and A. C. Dillon, 2010, Adv. Mater. 22, E145-49) reported high-rate capability binder-free electrode materials consist of $Fe_3O_4$ and single walled carbon nanotubes (SWCNTs). The best performance was obtained by the nanocomposite containing 95%wt oxide and 5%wt SWCNT. The nanocomposite was prepared by two-step process, hydrothermal synthesis and vacuum filtration. Most crucial step is the purification/deagglomeration of SWCNTs, which is time consuming. In addition, SWCNTs were synthesized by laser vaporization technique; both the material and method are very expensive.

Another solution for circumventing the problems of volume changes and agglomeration of $Fe_3O_4$ as anode material is proposed by Ji et al. (L. Ji, Z. Tan, T. Kuykendall, S. Aloni, S. Xun, E. Lin, V. Battaglia and Y. Zhang, 2011, Phys. Chem. Chem. Phys. 13, 7170-77). They describe a nanocomposite of reduced-graphene-oxide (RGO) in which $Fe_3O_4$ nanoparticles are encapsulated. The $Fe_3O_4$ nanoparticles are uniformly anchored in the RGO sheets such that severe volume changes or agglomeration of the particles can be avoided. The material was tested as anode material in Lithium ion batteries. It has to be noted that the synthesis of the $Fe_3O_4$ -RGO nanocomposite is a time consuming multi step process.

Wang et al. (J.-Z. Wang, C. Zhong, D. Wexler, N.H. Idris, Z.-X. Wang, L.-Q. Chen, H.-K. Liu, 2011, Chem. Eur. J., 17,661-667) have also prepared a nanocomposite consisting of $Fe_3O_4$ particles embedded in graphene nanosheets. The nanocomposite was tested as electrode material in a lithium ion battery where it exposed significantly improved life cycles compared to pure $Fe_3O_4$. The nanocomposite was synthesized in situ starting from graphite and $FeCl_2$ by a multistep hydrothermal method.

A further $Fe_3O_4$ nanocomposite in form of hierarchically structured $Fe_3O_4$/carbon micro-flowers has been prepared by Jin et al. (S. Jin, H. Deng, D. Long, X. Liu, L. Zhan, X. Liang, W. Qiao, L. Ling, 2011, J. Power Sources, 196, 3887-3893). This composite consists of nanosized $Fe_3O_4$ crystallites and amorphous carbon which are assembled to micro flowers of 2-5 $\mu$m. The paper also discloses a method of a controlled thermal decomposition of an iron-alkoxide precursor for preparing said nanocomposite. This preparation requires energy and time consuming efforts. The $Fe_3O_4$ nanocomposite is proposed and assayed for its capability as anode material in lithium ion batteries.

Panchal et al. disclose carbon coated $Fe_3O_4$ nanoparticles showing a core-shell structure. (V. Panchal, M. Neergat, U. Bhandarkar "Synthesis and characterization of carbon coated nanoparticles produced by a continuous low-pressure plasma process", 2011, J. Nanopart. Res., 13, 3825-3833). The particle size ranges from 15 to 24 nm whereas the thickness of the coating is around 2 to 5 nm. Carbon nanotubes are not present in the described material. The nanoparticles are prepared by a continuous low pressure plasma-based process wherein iron nanoparticles are first created starting from $Fe(CO)_5$, then trapped and finally coated with carbon in an argon plasma by switching on different precursors. A possible use as electrode material is not mentioned.

US 2011/0104553 A1 discloses carbon-coated metal oxide or metal phosphate nanoparticle material comprising particles having a titanium oxide core, a tin oxide core or a $LiMPO_4$ olivine core coated with about 0.5 to 30 percent by weight of carbon, the particles having a size in the range of 5 to 150 nm. The SiO2 and TiO2 particles have a size core size ranging from 5 to 20 nm with a carbon coating from 1 to 4 nm. The described materials are for use in an electrochemical cell. The particles are synthesized in an authogenic pressure reactor by heating metal oxalates, acetylacetonates or ethanolates to pyrolysis temperature. Carbon-coated magnetite particles are not described.

US 2010/0035750 A1 discloses a method for preparing carbon-coated metal oxide nanoparticles by performing at least the following two steps. A precursor of a polymer is polymerized on metal oxide nanoparticles in solution to perform polymer-coated metal oxide nanoparticles. Then, pyrolysis is conducted to carbonise the polymer so as to form the carbon-coated nanoparticles. The size of the obtained nanoparticles is about 15 nm. Carbon-coated magnetite particles or nanocomposites containing carbon nanotubes are not mentioned.

Tristao et al. describe a method for the preparation of' magnetic carbon coated $Fe_3O_4$ particles by a single step combined reduction of $Fe_2O_3$ together with a Chemical Vapor Deposition process using methane at temperatures between 600 and 900 °C to produce mainly $Fe_3O_4$ particles coated with up to 4 wt% of amorphous carbon (J. C. Tristao, A.A.S. Oliveira, J.D. Ardisson, A. Dias, R.M. Lago, Facile preparation of carbon coated magnetic Fe3O4 particles by a combined reduction/CVD process, 2011, Materials Research Bulletin 46(5), 748-754). The resulting carbon-coated magnetite particles have an average size of 100-200 nm. The authors propose a use of the particles in biomedicine; a use as electrode material in electrochemical cells is not disclosed.

A common disadvantage of the state of the art is the lack of an economic one-step solvent-free synthesis which is easy to perform. The cited state of the art does not disclose any nanoparticles or nanocomposites consisting of $Fe_3O_4$, which are encapsulated in a carbon shell.

## Objectives of the invention

To overcome the disadvantages of the state of the art, it is an objective of the present invention to provide a $Fe_3O_4$-carbon nanocomposite, which contains magnetite nanoparticles covered with a graphitic carbon onion shell.

It is another objective of the present invention to provide a simple one-step and solvent-free synthesis of said $Fe_3O_4$-carbon nanocomposite.

It is a further objective of the present invention to provide an anode material for its use in lithium-ion batteries. This anode material should be based on a $Fe_3O_4$-carbon nanocomposite, for reason of its good capacity, low toxicity and high electronic conductivity. The material should overcome the problems of kinetic limitations, agglomerations and poor cycling stability resulting from significant volume expansion during the redox reaction.

## Description of the invention

The invention provides a nanocomposite comprising carbon encapsulated magnetite nanoparticles and further carbon nanotubes. The nanoparticles have a core-shell structure, wherein a magnetite core with a diameter from 5 nm to 50 nm is covered by a graphitic carbon coating. Preferably the core diameter ranges from 10 nm to 30 nm.

Within the scope of the present invention, a core-shell structure shall read that the cores, which are formed by substantially spherically-shaped magnetite nanoparticles, are covered by a coating of several layers of graphitic carbon. This graphitic carbon shell has of a thickness from 1 nm to 5 nm. The interface between the carbon-coating and the magnetite is characterized by short-range disordered layers.

The nanocomposite comprises carbon nanotubes which are embedded in agglomerates of the carbon encapsulated $Fe_3O_4$ nanoparticles.

In a preferred embodiment, the nanocomposite consists of 70 to 90 wt% of $Fe_3O_4$ and 10 to 30 wt% of carbon in the form of graphitic carbon of the coating and of carbon nanotubes. The total composition of $Fe_3O_4$ and carbon does not exceed 100 wt%.

In another preferred embodiment the nanocomposite has a porous structure encompassing nanopores of a pore width from 0.1 nm to 2 nm and mesopores having sizes from 2 nm to 50 nm, more preferred between 10 nm and 35 nm. The BET (Brunauer, Emmett, Teller) specific surface area ranges preferably from 80 $m^2g^{-1}$ to 150 $m^2g^{-1}$.

The present invention provides a method to synthesize a carbon encapsulated $Fe_3O_4$ nanocomposite as described above. An oxygen-containing organic iron compound is submitted to pyrolysis in a hermetically closed vessel under inert gas atmosphere at a temperature from 500 to 800°C. Under these reaction conditions, any presence of organic oxygen is trapped by the iron to form an oxide.

The organic rests of the oxygen-containing organic iron compound, which mainly consist of carbon, are substantially reduced to the graphitic coating and to carbon nanotubes. In a preferred embodiment, these organic rests are alkoxides, carboxides or carbonyls. In a more preferred embodiment, the oxygen-containing organic iron compound is iron carbonyl $Fe(CO)_5$.

In another preferred embodiment, the reaction vessel is rotated during the reaction. This rotation allows the gentle mixing of the dry powder during the pyrolysis leading to a homogenous product. The reaction time is preferably from 1 to 3 hours.

One advantage of the provided method is that it can be performed in the absence of any solvent. Further additives such as surfactants are not required for this synthesis. In a preferred embodiment, the reaction mixture should contain no or minute amounts of reducing agents. Under certain conditions, the presence of such agents may lead to the formation of a mixture of transition metal and various transition metal oxides.

By applying the synthesis as described, a crude product is obtained which consists of a nanocomposite containing carbon encapsulated $Fe_3O_4$ nanoparticles having a core-shell structure as defined above and carbon nanotubes, which have formed in situ during the pyrolytic synthesis.

Another embodiment of the present invention is directed to an anode material for lithium-ion batteries containing a nanocomposite as defined herein. The $Fe_3O_4$ nanoparticles which are encapsulated within a shell of thin conductible graphitic carbon demonstrate good electrochemical performance. The short-range disordered layers of the interface

between the $Fe_3O_4$ core and the carbon shell facilitate the lithium ion transfer from and to the active material in the core.

In particular the network of the core-shell nanoparticles with the nanotubes confers an excellent electronic transport within the electrode. Moreover, the nanocomposite's morphology based on the core-shell structure and its porous structure gives the anode material good ionic transport capacities while the composite resists to volume changes and to agglomeration during the charge and discharge reactions.

Particularly advantageous is the use of the nanocomposite consisting of carbon-encapsulated $Fe_3O_4$ cores ($Fe_3O_4$ -C nanocomposite) as the iron compounds are abundant and nontoxic while having a good capacity and high electronic conductivity.

In a preferred embodiment the anode material further contains 1-10 wt % of carbon black and 1-10 wt % of PVDF-HFP (poly(vinylidene fluoride-co-hexafluoropropylene).

When using $Fe_3O_4$ in the carbon-encapsulated nanoparticles, the resulting nanocomposite shows magnetic activity. This magnetic nanocomposite can be used in different applications wherein the magnetic properties combined with the characteristics of the carbon-encapsulated $Fe_3O_4$ nanoparticles are suitable. These characteristics are e.g. no antiferromagnetic coupling between encapsulated $Fe_3O_4$ particles, the non-toxicity, or the chemical passivity of the carbon-shell structure.

For example the $Fe_3O_4$ -C nanocomposite can be used as therapeutic agent in medical applications. Upon exposure to a suitable electromagnetic field, the $Fe_3O_4$ -C nanocomposite can capture this field and transform it into heat. Upon local application of the nanocomposite into a solid tumor and exposing this area to an appropriate electromagnetic field, the neoplastic tissue can be destroyed non-invasively by heat.

Further magnetic applications of the carbon-encapsulated $Fe_3O_4$ nanoparticles, such as in ferrofluids are possible.

**Examples**

The following figures and examples are presented to provide a better understanding of the description of procedures and conceptual aspects of the invention.

**Fig. 1:** X-ray powder diffraction (XRD) pattern and Raman spectrum (inset) of $Fe_3O_4$-C nanocomposite.

**Fig. 2: a)** scanning electron microscope (SEM) and **b, c, d)** High resolution transmission electron microscopy (HRTEM) images of $Fe_3O_4$-C nanocomposite.

**Fig. 3:** Cumulative and differential pore width profile of $Fe_3O_4$-C nanocomposite.

**Fig. 4:** Charge/discharge curves of $Fe_3O_4$-C nanocomposite electrode.

**Fig. 5:** Cyclic voltammogram of $Fe_3O_4$-C nanocomposite electrode.

**Fig. 6:** Rate performance profile of $Fe_3O_4$-C nanocomposite electrode.

**Example 1: Synthesis of $Fe_3O_4$-C nanocomposite**

$Fe(CO)_5$ was sealed under argon atmosphere into a stainless steel Swagelok-type reactor. The reactor was placed horizontally inside a rotating quartz-tube setup in a furnace. The tube was rotated (10 rpm) during pyrolysis to obtain a homogeneous mixture. The reactor was heated at a rate of 5 °C min$^{-1}$ to 700 °C and kept at this temperature for 3 h. The reaction took place under autogenous pressure. After cooling down the reactor to ambient temperature naturally, the remaining pressure was released carefully. A dry fine black powder of $Fe_3O_4$ -C nanocomposite produced was collected and used directly without any further treatment.

**Example 2: Analysis of $Fe_3O_4$-C nanocomposite**

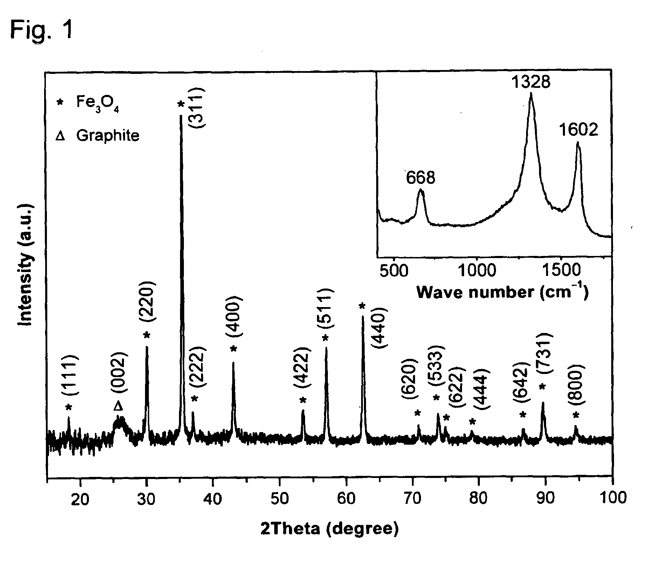

X-ray powder diffraction (XRD) pattern and Raman spectrum of the as prepared nanocomposite are shown in **Fig. 1.** All the diffraction peaks can be indexed to two well-defined phases, a hexagonal phase graphitic carbon [26.4° (002)] and a cubic phase $Fe_3O_4$. No detectable signals for metallic iron or other oxides were observed in the XRD pattern, indicating that the oxidation reaction was complete and formed only the selective oxide. The Scherrer analysis has been performed on 220, 311, 400, 511 and 440 Bragg peaks of $Fe_3O_4$, and the mean crystallite size was calculated as 14 nm.

The Raman spectrum of the composite exhibits two bands at 1328 and 1602 cm$^{-1}$, which are characteristic of the D (disorder-induced phonon mode15) and G (graphitic lattice mode $E_{2g}$[16]) bands of carbon, respectively. The intensity ratio $I_G/I_D$ of 0.7 indicated that a significant quantity of disordered carbon is also present in the nanocomposite. In addition, the $A_{1g}$ vibration mode of $Fe_3O_4$ peak appeared at 668 cm$^{-1}$, which agrees well with the literature value for pure as well as graphene encapsulated $Fe_3O_4$. The infra-red spectrum of the nanocomposite exhibits a broad band at 560 cm$^{-1}$ which is typical for the Fe-O vibration of $Fe_3O_4$.

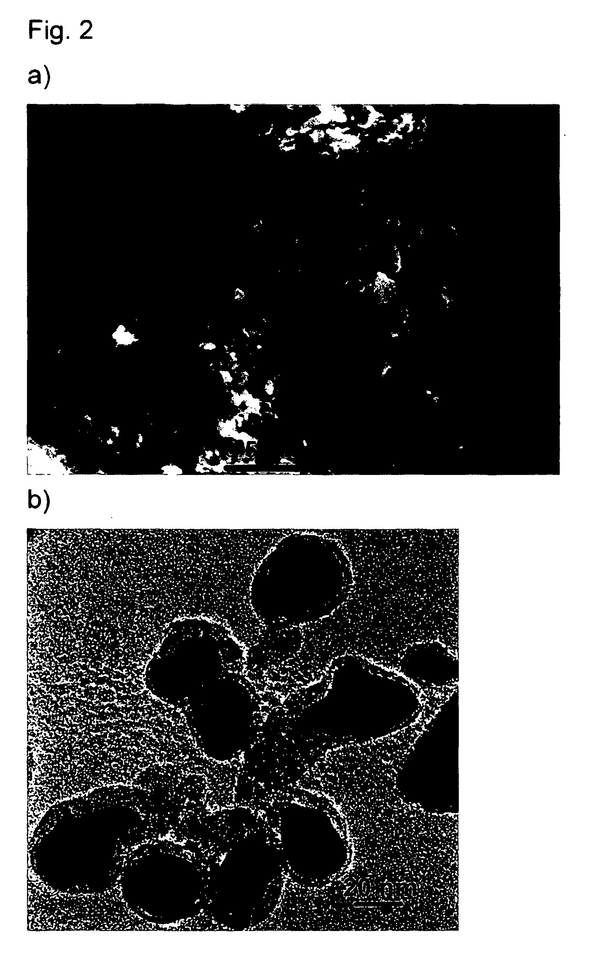

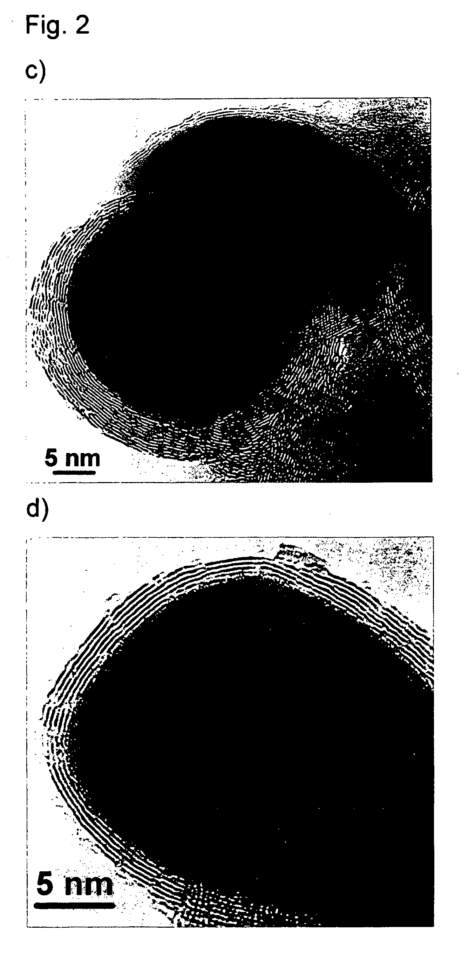

The scanning electron microscope (SEM) image of $Fe_3O_4$-C nanocomposite (**Fig. 2 a**) shows that the material consists of nanotubes and nanogranular structures interlinking with each other. The diameters of the tubes are in the range between 10 to 100 nm and their lengths varied up to several micrometers. A large number of tubes were encapsulated with iron oxide nanoparticles at their tips. However in some longer tubes, the particles were embedded in several places within the tube.

High resolution transmission electron microscopy (HRTEM) image (**Fig. 2 b, c, d**) of the nanogranular region of the composite confirmed the presence of core-shell structure, containing $Fe_3O_4$ cores and graphitic onions shells. The interface between graphitic carbon and $Fe_3O_4$ with short-range disordered layers can be observed. The $Fe_3O_4$ particles are coated with three to six layers of graphitic carbon with an average coating thickness of about 3 nm. However, a few $Fe_3O_4$ particles are surrounded by several layers of carbon.

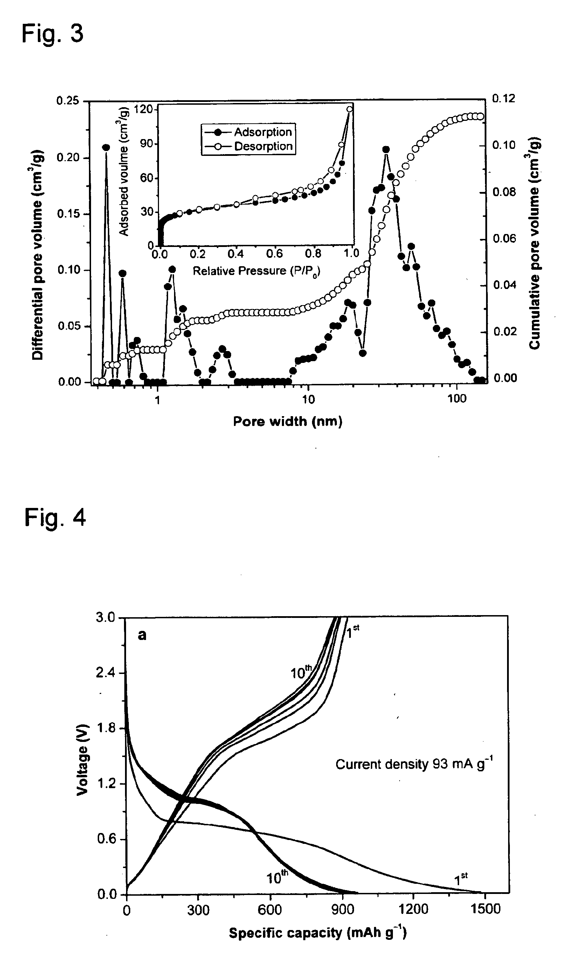

In **Fig. 3** the differential pore volume (filled circles) and the cumulative pore volume (open circles) are illustrated. The nitrogen adsorption-desorption measurement indicates mesoporous nature of the material. In addition, a sharp increase of the adsorbed gas at very low relative-pressures suggesting the presence of nanopores (pore width < 2 nm). The nano- and mesoporous volumes of were determined as 0.012 and 0.05 cm$^3$g$^{-1}$, respectively. The differential pore volume (filled circles in **Fig. 3**) estimated from the adsorption branch of isotherm using Density Functional Theory model suggests that the mesopore sizes were distributed between 10 and 35 nm. The BET (Brunauer, Emmett, Teller) specific surface area was calculated to be as high as 110.6 m$^2$g$^{-1}$ (open circles in **Fig. 3**).

It has been well established that porous electrode materials facilitated lithium ion diffusion to active sites with less resistance and also withstand the volume change during charge/discharge cycling. Thus, the nano- and mesopores of the $Fe_3O_4$ -C nanocomposite could act as buffer against the volume change during redox cycle, which would lead to an enhanced cyclic performance as an anode material for lithium ion batteries.

**Example 3: Electrochemical properties**

The electrochemical performance of the $Fe_3O_4$ -C nanocomposite anode has been evaluated with respect to lithium metal. **Fig. 4** shows a typical galvanostatic profile for a $Fe_3O_4$ -C nanocomposite cell cycled between 3 and 0.005 V at 93 mAg$^{-1}$. The obtained charge/discharge profiles are analogous to that of various $Fe_3O_4$ electrodes tested at similar current and voltage ranges. During the first discharge the potential dropped abruptly down to about 0.8 V, which can be ascribed to the reaction of $Fe_3O_4 + xLi \rightarrow Li_xFe_3O_4$. The obvious long plateau corresponds to the conversion reaction and the sloping part of the discharge curve can be assigned for the formation of solid electrolyte interface (SEI) layer, as well as gel-like film formed by the reaction of $Fe^0$ and electrolyte. The electrode exhibits a first discharge capacity of 1480 mAhg$^{-1}$ based on the total composite weight and the first charge capacity of 960 mAhg$^{-1}$. The capacity decreases marginally over the first few cycles and then stabilizes at about 920 mAhg$^{-1}$ in the subsequent 50 cycles. The coulombic efficiency after the first cycle was remained nearly 100% until 50 cycles.

The cyclic voltammogram of $Fe_3O_4$-C nanocomposite is parallel to that of other $Fe_3O_4$ electrodes, showing a cathodic wave at 0.56 V and an anodic wave at 1.78 V corresponding to $Fe^{3+}/Fe^{2+}$ to $Fe^0$ redox couple. The irreversible wave at 0.4 V can be ascribed to the SEI formation. In the subsequent cycles, the reversible waves shifted slightly to positive potentials. The CV curves after third successive scans nearly overlapped, which reveal the good reversibility of the composite electrode.

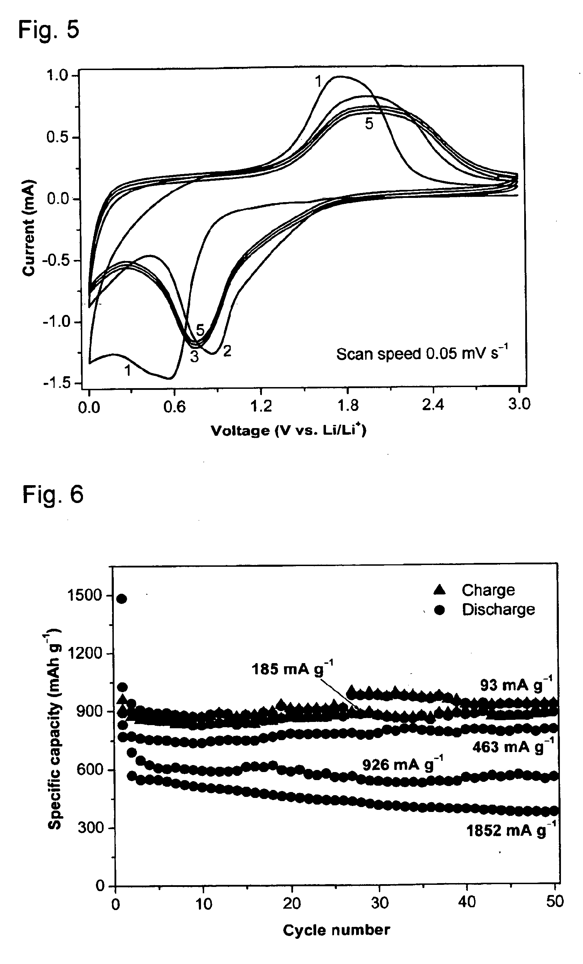

Besides the cyclic stability, it also exhibits a moderate rate capability performance (**Fig. 6**). Current densities below 926 mAg$^{-1}$, the electrode exhibited good cyclic performances and excellent capacity retention. When the electrode cycled at 1852 and 2780 mAg$^{-1}$, the observed capacity retentions between 3$^{rd}$ and 50$^{th}$ cycles were about 72 and 63%, respectively.

A comparative SEM image of the electrode cycled for 50 cycles at 93 mAg$^{-1}$ has shown similar morphology as of the original composite, thus indicating that the structure remains integrated during cycling. The good electrochemical performance of the composite can be attributed to its morphology and porous structure. Especially, the networking of nanotubes and core-shell structure containing active materials combined with nano and mesopores may help providing better electronic and ionic transport, as well as withstanding the volume change during the reaction.

**Claims**

1. A nanocomposite comprising carbon-encapsulated $Fe_3O_4$ nanoparticles with a core-shell structure, wherein the $Fe_3O_4$ core has a diameter from 5 nm to 50 nm which is covered by a graphitic coating of a thickness from 1 nm to 5 nm and wherein the nanocomposite further comprises carbon nanotubes embedded in the composite.

2. A nanocomposite according to claim 1, wherein the composite consists of 70 to 90 wt% of transition metal oxide and 10 to 30 wt% of carbon.

3. A nanocomposite according to claim 1 or 2, which comprises nanopores of a pore size from 0.1 nm to 2 nm and mesopores having a pore size from 2 nm to 50 nm.

4. A method to synthesize a carbon encapsulated $Fe_3O_4$ nanocomposite according to one of the claims 1 to 3 by pyrolysis of an oxygen-containing organic transition metal compound in a closed vessel under inert gas atmosphere at a temperature from 500 to 800°C.

5. A method according to claim 4, wherein the vessel is rotated during the reaction.

6. A method according to claim 4 or 5, wherein the reaction time is from 1 to 3 hours.

7. A method according to claim 4 to 6, wherein the oxygen-containing organic transition metal compound is $Fe(CO)_5$.

8. Anode material for lithium-ion batteries comprising a nanocomposite according to one of the claims 1 to 3.

9. Anode material according to claim 8 further comprising 1 to 10 %wt of carbon black and 1 to 10 %wt of PVDF-HFP (poly(vinylidene fluoride-co-hexafluoropropylene)).

**Patentansprüche**

1. Nanokomposit umfassend in Kohlenstoff eingeschlossene $Fe_3O_4$ Nanopartikel mit einer Kern-Hülle Struktur, wobei der Fe3O4 Kern ein Durchmesser von 5 nm bis 50 nm aufweist, der mit einer Graphit-Beschichtung mit einer Dicke von 1 nm bis 5 nm bedeckt ist und wobei das Nanokomposit weiterhin im Komposit eingeschlossene Kohlenstoffnanoröhrchen umfasst.

2. Nanokomposit nach Anspruch 1, wobei das Komposit 70 bis 90 Gew.-% Übergangsmetalloxid und 10 bis 30 Gew.-% Kohlenstoff enthält.

3. Nanokomposit nach Anspruch 1 oder 2, das Nanoporen mit einer Porengröße von 0,1 nm bis 2 nm und Mesoporen mit einer Porengröße von 2 nm bis 50 nm umfasst.

4. Verfahren zur Herstellung von einem Nanokomposit mit in Kohlenstoff eingeschlossenen $Fe_3O_4$ nach einem der Ansprüche 1 bis 3 mittels Pyrolyse einer Sauerstoff-enthaltenden organischen Übergangsmetall-Verbindung in einem geschlossenen Behälter bei einer Temperatur von 500 bis 800 °C unter Inertgas-Atmosphäre.

5. Verfahren nach Anspruch 4, wobei der Behälter während der Reaktion rotiert wird.

6. Verfahren nach Anspruch 4 oder 5, wobei die Reaktionsdauer 1 bis 3 Stunden beträgt.

7. Verfahren nach einem der Ansprüche 4 bis 6, wobei die Sauerstoff-enthaltende organische Übergangsmetall-Verbindung $Fe(CO)_5$ ist.

8. Anodenmaterial für Lithium-Ionen Batterien umfassend ein Nanokomposit nach einem der Ansprüche 1 bis 3.

9. Anodenmaterial nach Anspruch 8 weiterhin umfassend 1 bis 10 Gew.-% Carbon Black und 1 bis 10 Gew.-% PVDF-HFP (Polyvinylidenfluorid-co-hexafluorpropylen).

**Revendications**

1. Nano-composite renfermant des nanoparticules de $Fe_3O_4$ encapsulées dans du carbone ayant une structure noyau/coquille, dans lequel le noyau en $Fe_3O_4$ a un diamètre situé dans la plage de 5 à 50 nm qui est recouvert par un revêtement graphitique ayant une épaisseur située dans la plage de 1 à 5 nm, ce nano-composite renfermant en outre des nanotubes de carbone noyés dans le composite.

2. Nano-composite conforme à la revendication 1, consistant en 70 à 90 % en poids d'oxyde de métal de transition et 10 à 30 % en poids de carbone.

3. Nano-composite conforme à la revendication 1 ou 2, renfermant des nano-pores ayant une dimension située dans la plage de 0.1 à 2 nm et des méso-pores ayant une dimension située dans la plage de 2 à 50 nm.

4. Procédé de synthèse d'un nano-composite en $Fe_3O_4$ encapsulé dans du carbone conforme à l'une des revendications 1 à 3 par pyrolyse d'un composé organique d'un métal de transition renfermant de l'oxygène dans un réacteur fermé sous une atmosphère de gaz inerte à une température située dans la plage de 500 à 800°C.

5. Procédé conforme à la revendication 4, selon lequel le réacteur est mis en rotation au cours de la réaction.

6. Procédé conforme à la revendication 4 ou 5, selon lequel le temps de réaction est de 1 à 3 heures.

7. Procédé conforme à l'une des revendications 4 à 6, selon lequel le composé organique d'un métal de transition renfermant de l'oxygène est $Fe(CO)_5$.

8. Matériau d'anode pour des batteries lithium-ion renfermant un nano-composite conforme à l'une des revendications 1 à 3.

9. Matériau d'anode conforme à la revendication 8, renfermant en outre 1 à 10 % en poids de noir de carbone et 1 à 10 % en poids de PVDF-HPF (copolymère(fluorure de polyvinylidène-hexafluroropropylène)).

## Fig. 1

Fig. 2

a)

b)

Fig. 2

c)

5 nm

d)

5 nm

## Fig. 3

## Fig. 4

## Fig. 5

## Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110104553 A1
- US 20100035750 A1

**Non-patent literature cited in the description**

- **B. BANOV ; L. LJUTZKANOV ; I. DIMITROV ; A. TRIFONOVA ; H. VASILCHINA ; A. ALEKSANDROVA ; A. MOCHILOV ; B. T. HANG ; S. OKADA ; J. I. YAMAKI.** *J. Nanosci. Nanotech,* 2008, 591-94
- **H. LIU ; G. WANG ; J. WANG ; D. WEXLER.** *Electrochem. Commun.,* 2008, 1879-82
- **T. MURALIGANTH ; A.V. MURUGAN ; A. MANTHIRAM.** *Chem.Commun.,* 2009, 7360-62
- **S. WANG ; J. ZHANG ; C. CHEN.** *J. Power Sources,* 2010, vol. 195, 5379-81
- **P. LIAN ; X. ZHU ; H. XIANG ; Z. LI ; W. YANG ; H. WANG.** *Electrochim. Acta,* 2010, 834-40
- **S. SONG ; R. RAO ; H. YANG ; H. LIU ; A. ZHANG.** *Nanotechnology,* 2010, 185602
- **H. HE ; L. HUANG ; J. S. CAI ; X. M. ZHENG ; S. G. SUN.** *Electrochim. Acta,* 2010, vol. 55, 1140-44
- **M. ZHANG ; D. LEI ; X. YIN ; L. CHEN ; Q. LI ; Y. WANG ; T. WANG.** *J. Mater. Chem.,* 2010, vol. 20, 5538-43
- **G. ZHOU ; D.-W. WANG ; F. LI ; L. ZHANG ; N. LI ; Z.-S. WU ; L. WEN ; G. Q. LU ; H.-M. CHENG.** *Chem. Mater.,* 2010, vol. 22, 5306-13
- **C. BAN ; Z. WU ; D. T. GILLASPIE ; L. CHEN ; Y. YAN ; J. L. BLACKBRURN ; A. C. DILLON.** *Adv. Mater.,* 2010, vol. 22, E145-49
- **L. JI ; Z. TAN ; T. KUYKENDALL ; S. ALONI ; S. XUN ; E. LIN ; V. BATTAGLIA ; Y. ZHANG.** *Phys. Chem. Chem. Phys.,* 2011, vol. 13, 7170-77
- **J.-Z. WANG ; C. ZHONG ; D. WEXLER ; N.H. IDRIS ; Z.-X. WANG ; L.-Q. CHEN ; H.-K. LIU.** *Chem. Eur. J.,* 2011, vol. 17, 661-667
- **S. JIN ; H. DENG ; D. LONG ; X. LIU ; L. ZHAN ; X. LIANG ; W. QIAO ; L. LING.** *J. Power Sources,* 2011, vol. 196, 3887-3893
- **V. PANCHAL ; M. NEERGAT ; U. BHANDARKAR.** Synthesis and characterization of carbon coated nanoparticles produced by a continuous low-pressure plasma process. *J. Nanopart. Res.,* 2011, vol. 13, 3825-3833
- **J. C. TRISTAO ; A.A.S. OLIVEIRA ; J.D. ARDISSON ; A. DIAS ; R.M. LAGO.** Facile preparation of carbon coated magnetic Fe3O4 particles by a combined reduction/CVD process. *Materials Research Bulletin,* 2011, vol. 46 (5), 748-754